(19) Europäisches Patentamt
European Patent Office
Office européen des brevets

(11) Veröffentlichungsnummer: **0 231 444 A2**

(12) **EUROPÄISCHE PATENTANMELDUNG**

(21) Anmeldenummer: **86115427.6**

(22) Anmeldetag: **07.11.86**

(51) Int. Cl.⁴: **A61K 31/41**, C07D 249/18

Die Bezeichnung der Erfindung wurde geändert (Richtlinien für die Prüfung im EPA, A-III, 7.3).

(30) Priorität: **13.11.85 DE 3540149**

(43) Veröffentlichungstag der Anmeldung: **12.08.87 Patentblatt 87/33**

(84) Benannte Vertragsstaaten: **AT BE CH DE ES FR GB GR IT LI LU NL SE**

(71) Anmelder: **Boehringer Mannheim GmbH**
**Sandhofer Strasse 116**
**D-6800 Mannheim 31(DE)**

(72) Erfinder: **Bartsch, Wolfgang, Dr. med. vet.**
**Franconville-Strasse 5**
**D-6806 Viernheim(DE)**
Erfinder: **Simon, Herbert, Dr. rer. nat.**
**Händelstrasse 5**
**D-6840 Lampertheim(DE)**
Erfinder: **Woog, Heinrich, Dr. rer. nat.**
**Lindenstrasse 6**
**D-6941 Laudenbach(DE)**

(54) **Optisch aktive Isomere und Racemat von 1-(Benztriazol-4-yloxy)-3-[2-(2-methoxyphenoxy)-ethylamino]-2-propanol, Verfahren zu ihrer Herstellung sowie ihre Verwendung als Arzneimittel.**

(57) Verwendung von 1-(Benztriazol-4-yloxy)3-[2-methoxy-phenoxy)ethylamino]-2-propanol als Antiglaukommittel, optisch aktive R und S Isomere dieser Substanz, Verfahren zur Herstellung derselben sowie Arzneimittel, die diese Substanzen enthalten.

EP 0 231 444 A2

Xerox Copy Centre

**Verwendung von 1-(Benztriazol-4-yloxy)3-[2-(2-methoxyphenoxy)-ethylamino]-2-propanol als Antiglaukommittel, optisch aktive R und S Isomere dieser Substanz, Verfahren zur Herstellung derselben sowie Arzneimittel, die diese Substanzen enthalten**

Die Efindung betrifft die Verwendung von 1-(Benztriazol-4-yloxy)-3-[2-methoxyphenoxy)-ethylamino]-2-propanol als Antiglaukommittel, Arzneimittel, die diese Substanzen enthalten, die optisch aktiven R und S Isomeren dieser Substanz sowie Verfahren zur Herstellung derselben.

1-(Benztriazol-4-yloxyl)-3-[2-(2-methoxyphenoxy)-ethylamino]-2-propanol ist in der EP-B 14 951 als Beispiel 8 b in Form des racemischen Gemisches der R und S Isomeren beschrieben. Wie die übrigen Verbindungen dieser Patentschrift weist diese Verbindung ausgeprägte vasodilatierende Eigenschaften und eine Hemmung adrenergischer $\beta$-Rezeptoren auf, so daß sie zur Behandlung und Prophylaxe von Herz-und Kreislauferkrankungen empfohlen wird. Zwischenzeitlich durchgeführte weitere Untersuchungen mit der racemischen Form dieser Substanz haben ergeben, daß sie im Gegensatz zu den vorbekannten Verbindungen mit $\beta$-blockierender Wirkung vorwiegend eine $\beta_1$-blockierende Wirkung aufweist, d.h. die Rezeptoren des Herzmuskels blockiert und die $\beta_2$-Rezeptoren, d.h. die Rezeptoren der glatten Muskulatur nicht oder nur wenig beeinflußt. Diese Substanz erweist sich daher als "cardioselektiv". Die fehlende kontrahierende Wirkung auf die glatte Gefäß-und Bronchial-Muskulatur, durch die eine Reihe von Nebenwirkungen normaler $\beta$-blockierender Wirkstoffe vermieden werden, zusammen mit der vorteilhaften gefäß-erweiternden Wirkung, läßt diese Substanz für die Herz-Kreislauf-Therapie besonders interessant erscheinen.

Überraschenderweise wurde nun gefunden, daß die racemische Substanz ebenso wie die beiden Enantiomeren in extrem geringer Konzentration bei lokaler Applikation zur Senkung des Augeninnendrucks insbesondere in der Glaukomtherapie verwendet werden können.

Es ist zwar bereits seit langer Zeit bekannt, daß $\beta$-Rezeptoren-Blocker, wie Propranolol und andere Verbindungen, die eine Blockierung der $\beta_1$ und $\beta_2$ Rezeptoren aufweisen, bei der Glaukomtherapie den Augendruck senken (vergl. G.K.Kriegelstein, Klinische Monatsblätter für Augenheilkunde 172,677 ff (1978) jedoch zeigen sich bei der Lokalanwendung von $\beta$-Blockern dieses Typs unangenehme Nebenwirkungen, die zu brennenden und stechenden Schmerzen am Auge führten, sowie eine Oberflächenanaesthesie am Auge bewirken. Deshalb sind $\beta$-Blocker dieses Typs über Glaucomtherapie weniger geeignet.

Heute wird in großem Umfange Timolol (S-1-(t.Butylamino)-3-((4-morpholino-1,2,5-thiadiazol-3-yl)oxy)-2-propanol verwendet. Auch die Verwendung dieses Enantiomeren ergibt insbesondere bei der Langzeittherapie Nebenwirkungen, die teilweise auf $\beta_2$-Rezeptorenblockade zurückzuführen ist und eine Entwicklung trockener Augen (N.V.Nilsen, Z.Prakt. Augenheilkunde 2: 71 -77 (1981).

In der Europäischen Patentanmeldung 105 996 wird deshalb die Verwendung von R-Timolol bzw. einer an dem R-Isomeren angereicherten Mischung der Enantiomeren empfohlen, da die R-Form bei etwa gleich starker Senkung des Augendruckes eine wesentlich schwächere Wirkung auf die übrigen Organe aufweist als die S-Form, so daß Nebenwirkungen, wie eine Beeinflussung des Blutdruckes, der Herzfrequenz und insbesondere asthmatische Anfälle, die eine Folge der $\beta_2$-blockierenden Wirkung sind, weniger auftreten. In dieser Patentanmeldung sind eine Reihe weiterer $\beta$-blockierender Substanzen aufgeführt, von denen angenommen wird, daß sie ähnlich reagieren, ohne daß dafür ein experimenteller Nachweis angeführt ist.

In der Europäischen Patentanmeldung 114 048 wird weiterhin die Verwendung von D,L und D-Carazolol als Antiglaukom-Mittel empfohlen, da diese Verbindung aufgrund ihrer außerordentlich starken Wirkung nur in sehr geringer Dosierung angewendet werden muß (0,01 %-ige Lösung statt der üblichen 0,5 %-igen Lösung bei Timolol) so daß die systemischen Nebenwirkungen aufgrund der verringerten Dosierung nicht oder nur vermindert beobachtet werden.

Nachdem unsere Untersuchungen ergeben hatten, daß die erfindungsgemäße Substanz zwar eine $\beta_1$-blockierende Wirkung besitzt, jedoch nur eine nur sehr geringe $\beta_2$-blockierende Wirkung, schien es von Interesse, festzustellen, ob diese Substanzen auch den Augeninnendruck senken und somit als Glaukommittel einzusetzen sind oder ob diese Wirkung ebenfalls entfallen ist. Es stellte sich heraus, daß die augeninnendrucksenkende Wirkung im gleichen Maße vorhanden ist wie bei üblichen bekannten $\beta$-blockierenden Verbindungen, beispielsweise Timolol und Metipranolol, so daß diese Substanz, die aufgrund der fehlenden $\beta_2$-blockierenden Wirkung die sonst bekannten Nebenwirkungen nicht aufweist, vorteilhaft auch in der Glaukomtherapie eingesetzt werden kann. Nachdem "klassische" Beta-Blocker, wie z.B. Timolol, als weiteren Nebeneffekt den peripheren Gefäßwiderstand erhöhen und somit bei lokaler Applikation am Auge eine Durchblutungsminderung im Bereich des Sehnerves und eine Verschlechterung des

Kammerwasserabflußvermögens bewirken (H.J. Merté et al., Present Day Possibilities of Glaucoma Therapy: Pharmacology of Beta-blocking Agents and Use of Metipranolol in Ophthalmology, Springer-Verlag Wien/New York, Seite 22-34 (1983)) stellt die durchblutungsfördernde Wirkung der erfindungsgemäßen Verbindung einen weiteren Therapievorteil gegenüber klassischen Beta-Blockern dar.

Es wurde weiterhin gefunden, daß die R-und S-Enantiomeren der erfindungsgemäßen Substanz sich in ihren Wirkungen unterscheiden, insbesondere das S-Enantiomere praktisch die gesamte $\beta_1$-blockierende Wirkung sowie eine blutgefäßerweiternde und augendrucksenkende Wirkung aufweist, während das R-Enantiomere die vasodilatierende Wirkung mit der augendrucksenkenden Wirkung vereint, jedoch keine $\beta_1$-blockierende und auch keine $\beta_2$-blockierende Wirkung besitzt. Das R-Enantiomere kann daher mit Vorteil in der Glaukomtherapie auch in den Fällen eingesetzt werden, in denen die $\beta_1$-blockierende Wirkung auch in den geringen Konzentrationen, wie sie bei einer lokalen Applikation anfallen, stört.

Die S-und R-Enantiomeren wurden bisher nicht in Substanz beschrieben, so daß sie selbst und Verfahren zu Ihrer Herstellung einen weiteren Gegenstand dieser Erfindung bilden.

Die Enantiomeren können in üblicher Weise durch Diastereomerenbildung mit optisch aktiven Säuren und fraktionierte Kristallisation der entstehenden Salze gewonnen werden, jedoch ist dieses Verfahren mühsam und langwierig, da die Diastereomeren zu einer Mischkristallbildung neigen, so daß die optische Aktivität durch jeden Kristallisationsschritt nur wenig verbessert wird. Vorteilhafterweise können daher die Enantiomeren durch Animpfen mit den entsprechenden Isomeren gewonnen werden - (Schaukelracematentrennung) was jedoch voraussetzt, daß man über hochreine Impfkristalle der enantiomeren Substanz verfügt.

Bevorzugt wird daher eine asymmetrische Synthese, welche in hoher optischer Reinheit die beiden Enantiomere ergibt. Erfindungsgemäß wird dabei 4-Hydroxy-benztriazol der allgemeinen Formel I, welches durch eine Tetrahydropyranylgruppe geschützt ist,

(I) 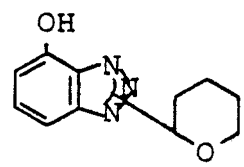

mit R-bzw. S-Glycidol der Formel IIa bzw. IIb

(IIa) 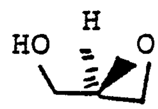

(R)

(IIb) 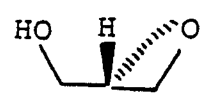

(S)

umgesetzt und das entstandene 1-(N-(2-Tetrahydropyranyl)benztriazol-4-yloxy)-2,3-epoxypropan der Formel IIIa/b

(IIIa) 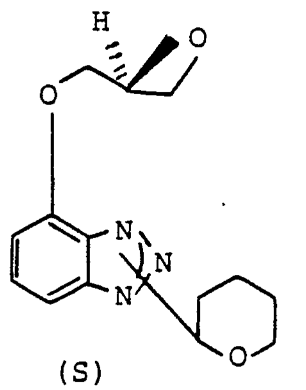

(S)

(IIIb) 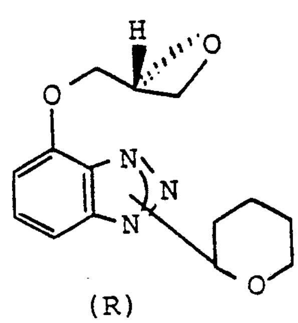

(R)

mit N-Benzyl-2-(2-methoxyphenoxy)ethylamin der Formel IV

(IV) 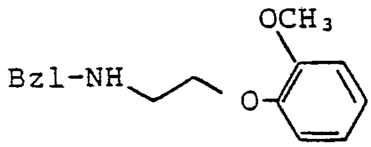

umgesetzt und aus dem erhaltenen 1-(Tetrahydropyranyl-(benztriazol-4-yloxy))-3-(N-benzyl-2-(2-methoxy-phenoxy)ethylamino)-2-propanol Va/b

(Va) 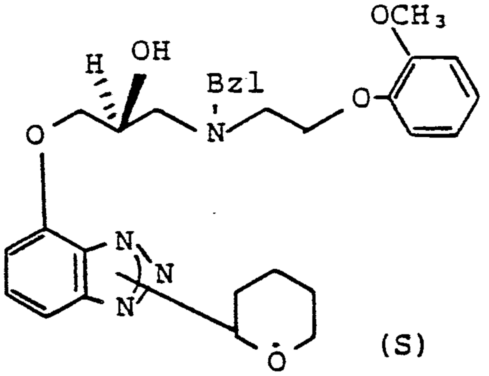

(S)

(Vb) 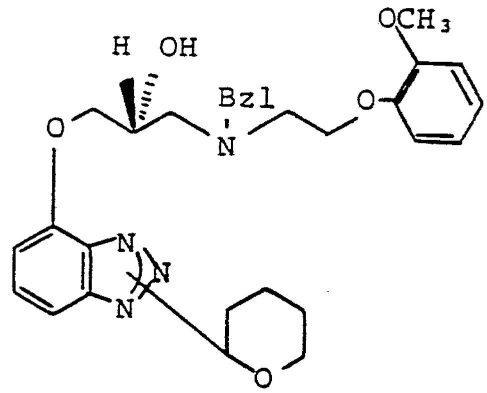

(R)

die Schutzgruppen abgespalten, wodurch die enantiomeren Verbindungen VIa und VIb erhalten werden.

(VIa) 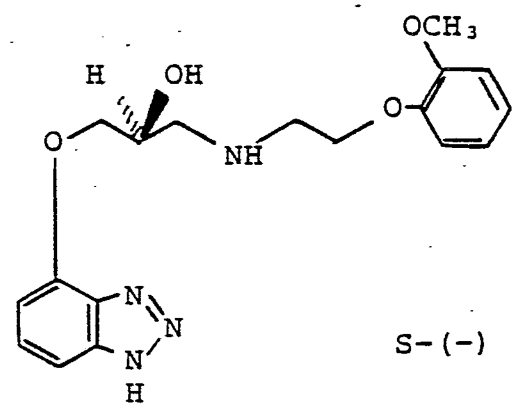

S-(-)

(VIb) 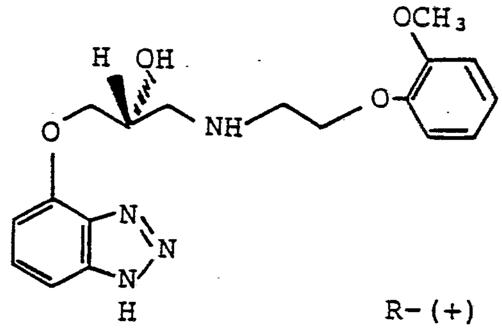

R-(+)

Die Herstellung der Ausgangsverbindungen S-Glycidol und R-Glycidol ist aus Jung et al., J.Am.Chem.Soc. 102, 6304 (1980) bzw. Baldwin et al., J.org.Chem. 43, 4876 (1978) bzw. der EP-A 127,099 bekannt. Die Veretherungsreaktion erfolgt in Analogie zu O.Mitsunobu, Synthesis (1981), 1 ff.

Die Umsetzung von I mit II erfolgt üblicherweise in inerten Lösungsmitteln wie Toluol, Benzol, Dimethylsulfoxid oder Methylenchlorid, wobei die Ausgangsverbindungen bei Raumtemperatur oder erniedrigter Temperatur vermischt werden und erst nachdem die Hauptreaktion abgeklungen ist, aufgewärmt und bei Raumtemperatur oder erhöhter Temperatur die Reaktion zuende gebracht wird. Das Zwischenprodukt der Formel III a bzw. b kann als solches isoliert werden, wird jedoch üblicherweise durch Eindampfen der Lösung und wieder Aufnehmen in einem polaren Lösungsmittel direkt mit dem Ethylamin der Formel IV weiter umgesetzt. Als polares Lösungsmittel wird in der Regel ein organisches Lösungsmittel wie Methanol, Ethanol, Isopropanol o.Ä. eingesetzt, in dem die Verbindungen bei erhöhter Temperatur bis zum Siedepunkt zur Umsetzung gebracht werden.

Die Abspaltung der Schutzgruppen aus den Verbindungen der Formel V a bzw. b erfolgt in üblicher Weise durch Hydrolyse mit wässriger Säure und Hydrogenolyse der Benzylgruppe mit molekularem Wasserstoff und Palladium auf Kohle als Katalysator. Wiederum können die Verbindungen zwischendurch gereinigt oder direkt weiter umgesetzt werden.

Nach dem vorstehenden Verfahren fallen die beiden Antipoden in einer optischen Reinheit von nahezu 100 % an, so daß sie ohne weiteres Aufkonzentrieren eingesetzt werden können.

Zur Glaukombehandlung werden die erfindungsgemäße racemische Substanz bzw. das R-Enantiomere als freie Base, vorzugsweise jedoch in Form ihrer pharmakologisch unbedenklichen Salze in Form von Augentropfen verwendet. Bevorzugt sind Salze mit physiologisch verträglichen anorganischen oder organischen Säuren, z.B.,Salzsäure, Bromwasserstoffsäure, Phosphorsäure, Schwefelsäure, Essigsäure, Salicylsäure, Citronensäure, Benzoesäure, Naphthoesäure, o-Acetoxybenzoesäure, Adipinsäure oder Maleinsäure.

Als Lösungsmittel kommt vorzugsweise eine isotone wässrige Lösung mit einem pH-Wert zwischen 5,8 und 7,5, vorzugsweise etwa 7.0 in Frage. Zur Einstellung des pH-Wertes wird der Lösung eine Puffersubstanz, beispielsweise Natrium-oder Caliumcitrat oder Phosphat oder Carbonat zugesetzt. Die Isotonie wird durch Zugabe von Natrium-oder Kaliumchlorid oder einer anderen, pharmakologisch verträglichen, das Auge nicht reizenden Substanz eingestellt.

Weiterhin kann die Lösung ein Konservierungsmittel wie Benzylalkohol, Benzalkoniumchlorid, Chlorcresol, Chlorbutanol oder Chlorhexidin enthalten. Als Lösungsvermittler bzw. viskositätsregulierende Mittel können ferner Polyethylenglycol, Polyvinylpyrrolidon oder Glycerin oder ähnliche Stoffe enthalten sein.

Die racemische Substanz bzw. das entsprechende R-Enantiomere kann zur Behandlung von Glaukom-Patienten in einer Konzentration von 0.1 bis 1 % eingesetzt werden. Die Augentropfen werden hierzu ein-oder mehrfach, vorzugsweise zweimal pro Tag appliziert.

Bevorzugt wird eine 0,2 bis 0,5 %-ige Lösung, die keine Nebenwirkungen, insbesondere kein anästhetisches Gefühl oder Brennen am Auge selbst oder asthmatische Anfälle o.Ä. bewirkt. Am Glaukompatienten dürftein der Regel jeweils ein Tropfen pro Applikation und Auge genügen, jedoch können insbesondere bei niederer konzentrierten Lösungen auch 2 bis 4 Tropfen appliziert werden.

Die nachfolgenden Beispiele beschreiben die Herstellung der Enantiomeren sowie einer repräsentativen Applikationsform von Augentropfen für die Glaukomtherapie.

<u>Beispiel 1</u>

R-(+)-1-(Benztriazol-4-yloxy)-3-2-(2-methoxyphenoxy)-ethylamino)-2-propanol-hydrochlorid

Zu einer Lösung von 16.7 g 4-Hydroxy-N-(2-tetrahydropyranyl)-benztriazol und 19.4 g Triphenylphosphin in 200 ml trockenem Toluol tropft man bei 8°C eine Lösung von 5.5 g (S)-Glycidol in 25 ml trockenem Toluol. Dann werden innerhalb 30 min bei 8 -10°C 11.7 ml Azodicarbonsäure-diethylester zugetropft. Man rührt noch 2 h bei 8 -10°C, dann 15 h bei Raumtemperatur. Die Lösung wird zur Trockne eingedampft: Rückstand (R)-1-(N-(2-Tetrahydropyranyl)benztriazol-4-yloxy)-2,3-epoxypropan (54 g).

54 g des so erhaltenen Produktgemisches und 15.1 g N-Benzyl-2-(2-methoxyphenoxy)ethylamin in 400 ml Ethanol werden 4 h zum Rückfluß erhitzt. Hierauf wird zur Trockene eingedampft und der Rückstand an Kieselgel chromatographiert (Eluierungsflüssigkeit Heptan/Essigester 1:3).
Man erhält 25.0 g (R)-1-(N-(2-Tetrahydropyranyl)benztriazol-4-yloxy)-3-(N-benzyl-2-(2-methoxyphenoxy)-ethylamino)-2-propanol (ölig).

25.0 g (R)-1-(N-(2-Tetrahydropyranyl)benztriazol-4-yloxy)-3-(N-benzyl-2-(2-methoxyphenoxy)-ethylamino)-2-propanol werden in 750 ml Ethanol gelöst, mit 50 ml 2N-Salzsäure und 10 g Pd/C (10%) versetzt und bei 40°C bis zur Beendigung der Wasserstoffaufnahme hydriert. Man filtriert vom Katalysator ab, versetzt mit 20 ml 6 N-Salzsäure und erwärmt für 15 Minuten. Daraufhin wird zur Trockne eingedampft, der Rückstand in 25 ml Ethanol heiß gelöst und die Lösung bis zur beginnenden Trübung mit heißem Essigester versetzt. Man läßt 15 h stehen, saugt ab und trocknet.

Ausbeute: 12.5 g (65.7 % d.Th.) R-(+)-1-(Benztriazol-4-yloxy)-3-(2-(2-methoxyphenoxy)-ethylamino)-2-propanol-hydrochlorid
Schmp.: 122°C
$(\alpha)_D^{20}$: + 9.35° (c = 1, Eisessig)
optische Reinheit: 97.8 % (HPLC)
chemische Reinheit: 96.3 % (HPLC)

Das als Ausgangsprodukt verwendete (S)-Glycidol wird wie folgt hergestellt:
35.5 g (S)-α,β-Isopropylidenglycerin-γ-tosylat werden mit 124 ml 1 N-Salzsäure und 75 ml Aceton versetzt und 40 min bei 80°C gerührt. Man dampft zur Trockne ein, löst den Rückstand in 150 ml Methylenchlorid und trocknet über Natriumsulfat. Man filtriert und dampft ein. Es verbleiben 29.9 g (S)-3-Tosyloxy-1,2-propandiol (98 % d.Th.) als öliger Rückstand. Das Ö1 wird in einer Mischung von 26.6 ml trockenem

Methanol und 50 ml trockenem Ether aufgenommen und bei 0 bis 5° C innerhalb einer Stunde eine Natriummethylat-Lösung (hergestellt aus 2.65 g Natrium und 60 ml trockenem Methanol) zugetropft. Man rührt noch 2 h bei 0 bis 5°C, saugt ab und engt das Filtrat bei 20°C unter vermindertem Druck ein. Der Rückstand wird in 100 ml Ether gelöst, die Lösung mit etwas Filtrierflocken behandelt, vom Feststoff abgesaugt und das Filtrat bei 20°C und vermindertem Druck eingedampft. Es verbleiben 5.5 g (S)-Glycidol (65 % d.Th.) als öliger Rückstand.

Beispiel 2

S-(-)-1-(Benztriazol-4-yloxy)-3-(2-(2-methoxyphenoxy)-ethyl-amino)-2-propanol-hydrochlorid

Zu einer Lösung von 34.0 g 4-Hydroxy-N-(2-tetrahydropyranyl)benztriazol und 39.6 g Triphenylphosphin in 400 ml trockenem Toluol tropft man bei 8°C eine Lösung von 11.2 g (R)-Glycidol in 50 ml trockenem Toluol. Dann werden innerhalb 30 min bei 8 -10°C 23.8 ml Azodicarbonsäure-diethylester zugetropft. Man rührt noch 2 h bei 8 -10°C, dann 15 h bei Raumtemperatur. Die Lösung wird zur Trockne eingedampft und der Rückstand (S)-1-(N-(2-Tetrahydropyranyl)benztriazol-4-yloxy)-2,3-epoxypropan (111.0 g) direkt weiter umgesetzt.

111.0 g des so erhaltenen Produktgemisches werden analog Beispiel 1 mit 30.7 g N-Benzyl-2-(2-methoxyphenoxy)ethylamin umgesetzt. Man erhält 45.6 g (S)-1-(N-(2-Tetrahydropyranyl)benztriazol-4-yloxy)-3-(N-benzyl-2-(2-methoxyphenoxy)ethylamino)-2-propanol (68.5 % d.Th.) als ölige Substanz.

45.6 g (S)-1-(N-(2-Tetrahydropyranyl)benztriazol-4-yloxy)-3-(N-benzyl-2-(2-methoxyphenoxy)-ethylamino)-2-propanol werden analog Beispiel 1 behandelt.

Ausbeute: 22.5 g (61.6 % d.Th.) S-(-)-1-(Benztriazol-4-yloxy)-3-(2-(2-methoxyphenoxy)-ethylamino)-2-propanol-hydrochlorid
Schmp.: 118°C
$(\alpha)_D^{20}$ : -7.95° (c = 1, Eisessig)
optische Reinheit: 99.6 % (HPLC)
chemische Reinheit: 85.4 % (HPLC).

Beispiel 3

Augentropfen

In 2000 ml Wasser werden 260 mg Trinatriumcitrat x $2H_20$, 27,12 g Natriumchlorid und 300 g Benzalkoniumchlorid gelöst. Anschließend wird die so gewonnene Lösung auf 50 -60°C erwärmt und darin 15 g 1-(Benztriazol-4-yloxy)-3-(2-(2-methoxyphenoxy)-ethylamino)-2-propanol oder das optisch aktive R-Isomere dieser Substanz gelöst.

Man läßt abkühlen auf Raumtemperatur, füllt mit Wasser auf das Endvolumen von 3000 ml auf und rührt um. Diese Lösung wird unter aseptischen Bedingungen über ein Membranfilter mit einer mittleren Porenweite von 0,2 µm steril filtriert.

10 ml dieser so gewonnenen Lösung werden unter aseptischen Bedingungen in Augentropfenflaschen abgefüllt.

**Ansprüche**

1.) Racemisches 1-(Benztriazol-4-yloxy)-3-(2-(2-methoxyphenoxy)-ethylamino)-2-propanol sowie seine pharmakologisch unbedenklichen Salze zur Verwendung bei der Glaucombehandlung.

2.) Verwendung von racemischen 1-(Benztriazol-4-yloxy)-3-(2-(2-methoxyphenoxy)-ethylamino)-2-propanol und seinen pharmakologisch unbedenklichen Salzen zur Glaukombehandlung.

3.) R-(+) und S-(-)-1-(Benztriazol-4-yloxy)-3-(2-(2-methoxy-phenoxy)-ethylamino)-2-propanol sowie ihre pharmakologisch unbedenklichen Salze.

4.) Verfahren zur Herstellung von optisch aktivem R-oder S-1-(Benztriazol-4-yloxy)-3-(2-(2-methoxyphenoxy)-ethylamino)-2-propanol sowie deren pharmakologisch verträglichen Salzen, dadurch gekennzeichnet, daß man 4-Hydroxy-N-(2-tetrahydropyranyl)benztriazol der Formel I

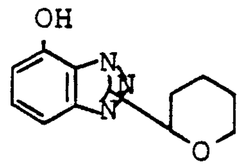

mit R-bzw. S-Glycidol der Formel IIa bzw. IIb

(IIa) 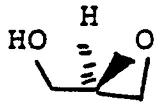

(R)

(IIb) 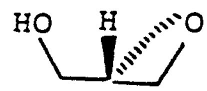

(S)

umsetzt und das entstandene 1-(N-(2-Tetrahydropyranyl)benztriazol-4-yloxy)-2,3-epoxypropan der Formel IIIa/b

(IIIa) 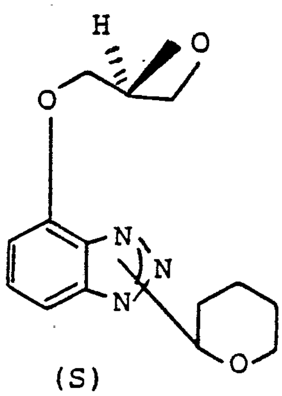

(S)

(IIIb) 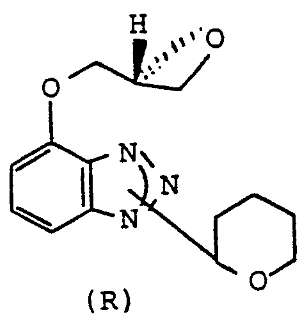

(R)

mit N-Benzyl-2-(2-methoxyphenoxy)ethylamin der Formel IV

(IV) 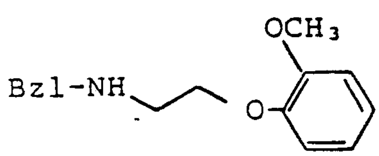

umsetzt und aus dem erhaltenen 1-(Tetrahydropyranyl-(benztriazol-4-yloxy))-3-(N-benzyl-2-(2-methoxy-phenoxy)ethylamino)-2-propanol Va/b

(Va) 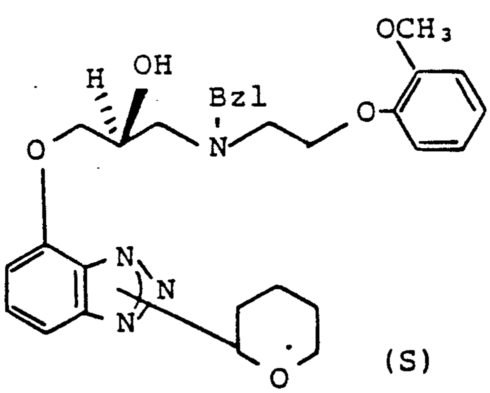

(S)

(Vb) 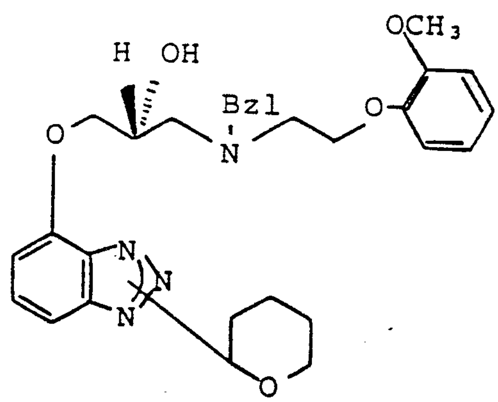

(R)

die Schutzgruppen abspaltet, wodurch die enantiomeren Verbindungen VIa und VIb erhalten werden

(VIa)

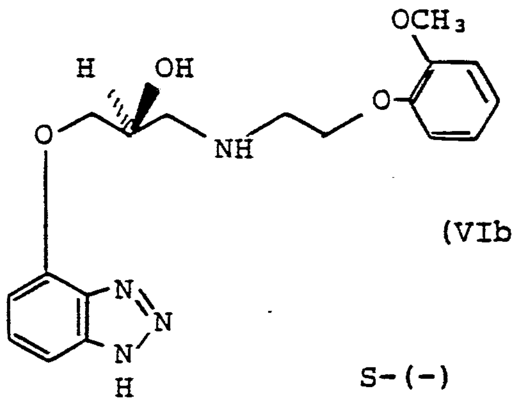

(VIb)

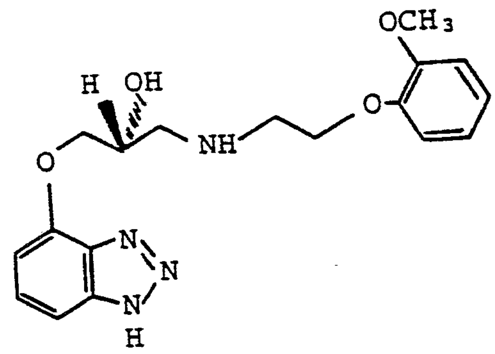

S-(-) R-(+)

und diese gewünschtenfalls durch Umsetzen mit den entsprechenden Säuren in die pharmakologisch verträglichen Salze überführt.

5.) Verwendung von Verbindungen gemäß Anspruch 3 zur Herstellung von Arzneimitteln.

6.) Verwendung von R-(+)-1-(Benztriazol-4-yloxy)-3-(2-(2-methoxy-phenoxy)-ethylamino)-2-propanol und seinen pharmakologisch unbedenklichen Salzen zur Glaukombehandlung.

7.) Augentropfen, enthaltend R,S-oder R-(+)-1-(Benztriazol-4-yloxy)-3-(2-(2-methoxyphenoxy)-ethylamino)-2-propanol bzw. ihre Salze und übliche Lösungsmittel,Puffer und Konservierungsstoffe.

8.) Augentropfen gemäß Anspruch 7 mit einer Wirkstoffkonzentration von 0,1 bis 1 %, vorzugsweise 0,2 -0,5 %.

9.) Verwendung von Verbindungen gemäß Anspruch 3 zur Herstellung von Augentropfen.

10.) Verwendung von S-(-) 1-(Benztriazol-4-yloxy)-3-(2-(2-methoxy-phenoxy)-ethylamino)-2-propanol und seinen pharmakologisch undedenklichen Salzen zur Behandlung von Herz-und Kreislauferkrankungen.